# EUROPEAN PATENT APPLICATION

(11) **EP 3 130 335 A1**
(43) Date of publication of application: **15.02.2017**
(21) Application number: 16183258.9
(22) Date of filing: 08.08.2016
(51) Int. Cl.: A61K 31/047, A61K 31/717, A61K 9/00, A61K 9/20, A61P 27/04, A61P 27/10

(54) **OPHTHALMIC COMPOSITION COMPRISING INOSITOL FOR IMPROVING VISUAL ACCOMODATION**

(30) Priority: 13.08.2015 IT UB20153104
(71) Applicant: Sooft Italia S.p.A., 63833 Montegiorgio (FM) (IT)
(72) Inventor: Biondi, Marco, 63833 Montegiorgio (FM) (IT); Biondi, Piero, 63833 Montegiorgio (FM) (IT); Stagni, Edoardo, 63833 Montegiorgio (FM) (IT); Stagni, Marcello, 63833 Montegiorgio (FM) (IT)
(74) Representative: Sarpi, Maurizio

(57) **Abstract**

An ophthalmic composition for topical use comprising myo-inositol, optionally in combination with hypromellose, as a therapeutic agent, for use in the treatment of eye disorders caused by environmental, mechanical and visual stress, including accommodative asthenopia and effort and to prolong the effect of therapy by electrostimulation of the ciliary muscle to counter presbyopia.

## Description

### Technical field

The present invention relates to the medical-pharmaceutical field, and more precisely to the ophthalmic preparations for topical use (eye drops) instilled on the corneal surface to be used as moisturizing tear substitutes indicated for the treatment of eye disorders caused by environmental, mechanical, visual stress including eye strain and visual accommodative effort.

### Background of the invention

Nowdays the work requirements and the new habits related to free time are leading more and more to an increase in the time spent in front of a screen or display of a computer, smartphone or to a video. The resulting ocular overload may lead to a real visual fatigue syndrome, also called asthenopia.

Asthenopia, or eye strain, is a visual disorder due to a work overload of the eyes. More specifically, such tiredness is due to the excessive effort of the intrinsic and extrinsic muscles of the eyeball. In fact, while looking at an object, such muscles are committed to focus (visual accommodation). Eye strain mostly affects people subjected to visual stress and those affected by visual defects not appropriately corrected, such as hyperopia, astigmatism, myopia, strabismus, amblyopia, blepharitis, conjunctivitis, dry eye syndrome and degenerative retinopathy.

Eye strain may be associated with burning, pain, and dry eye, photophobia, blurred or double vision and headaches, conjunctival redness, difficulty in reading, feeling of heaviness in the eyelids, sensitivity to light and need to keep eyes closed; such symptoms can then subsequently produce altered tear film, resulting in dry eye syndrome, headache, nausea, dizziness and low vision.

The visual accommodation, or accommodative process, is an autonomous mechanism of the visual apparatus, occurring by the increase of the curvature of the anterior lens surface through the ciliary muscle, this allows the focus of the images on the retina at different distances with respect to a remote point. The accommodation process starts from an initial blurred image, whose the brain is not satisfied. The process is as fast as the information transmission rate of the nerve cells allows it, independently on the subject's willing.

Visual accommodation disturbances commonly are refractive disorders, usually caused by anatomical changes such as nearsightedness or astigmatism, or by physiological aging as hyperopia. But they can also be of functional nature, particularly when they are caused by medications. Typically these disorders disappear at the end of the treatment. All drugs primarily acting on the autonomic nervous system may also affect the eyesight, other drugs can have unpredictable effects, sometimes involving hydration changes of ocular structures. Medicines containing substances acting on the autonomic nervous system may alter both accommodation and pupil size, as atropinic medications (anticholinergics), for example atropine and its derivatives hinder visual accommodation and cause mydriasis.

In the prior art several pharmacological aids based on saccharides (mono-, di-, oligo-, polysaccharides), alone or in combination with other active ingredients of various nature, solving eye disorders caused by environmental, mechanical and visual stress are known. The international patent application WO 2008/077110 describes ophthalmic solutions, in particular solutions for the cleaning, rinsing and maintaining contact lenses, as well as solutions for the delivery of active ingredients in the eye and the cleaning of ophthalmic devices, comprising from 0.00001 to 0.001% by weight of a simple disaccharide selected from: inositol, mannitol, sorbitol, ribose, triose, tetrose, erythrose, threose, pentose, arabinose, ribulose, xylose, xylulose, hexose, allose, altrose, fructose, sucrose, dextrose, galactose, glucose, gulose, idose, mannose, sorbose, talose, tagatose, adlose, ketose, eptose sedoeptulose, glycerine, xylitol and polyol, at least 0.00001% by weight of a preservative agent, and not more than 0.2% by weight of sodium chloride.

The European patent EP0585896 claims the use of a polyhydroxy alcohols selected among: sorbitol, mannitol, inositol, xylitol, and mixtures thereof; preferably sorbitol is at a concentration of 5% by weight, as active substance for the preparation of an ophthalmic composition for topical application to reduce elevated intraocular pressure of aqueous humor.

Some ophthalmic compositions comprise in their formulation inositol as an excipient, that is, an auxiliary substance for the production of the pharmaceutical form, it has to be pharmacologically inert and having low chemical reactivity, with the purpose to provide the composition with the form, the texture, the dilution and other physical and chemical-physical necessary features, while acting especially as a vehicle for active substances.

Although several ophthalmic products are available for the treatment of abnormal weakness of the eyes, due to the sight excessive strain or to visual defects, such as astigmatism and hyperopia, it continues to be high the demand of new compositions, having simpler formulation and proving to be even more effective in bringing the eyes in a state of rest, rehydrating the tear film, restoring the physiological conditions, and decongesting both eyelids and conjunctiva, thereby reducing the symptoms arising from excessive visual strain.

### Summary of the invention

It is an object of the present invention an ophthalmic composition comprising inositol for the treatment of eye disorders caused by environmental, visual and mechanical stress.

In a particularly preferred embodiment of the invention the ophthalmic composition is further comprising inositol and hydroxypropylmethylcellulose for the treatment of eye disorders caused by environmental, visual and mechanical stress.

The ophthalmic compositions of the invention are particularly useful for improving eye accommodative amplitude in patients undergoing electrical stimulation of the ciliary muscle to treat presbyopia.

### Detailed description of the invention

The present invention relates to a pharmaceutical composition formulated for ophthalmic topical administration, whose fundamental and characteristic component is inositol, preferably in its isomeric form of myo-inositol.

Inositol is a carbocyclic polyol, whose molecule is present in nature in several chemical forms (isomers), for example, scyllo-inositol, chiro-inositol, neo-inositol, allo-inositol, epi-inositol, etc., but the most common isomer is myo-inositol.

Inositol plays an important role in various cellular biological processes as a secondary messenger, in the form of inositol phosphate, as phosphatidylinositol (PI) or phosphatidylinositolphosphate (PIP).

In particular, the myo-inositol takes part in important biological processes, such as morphogenesis and cytogenesis, lipid synthesis, formation of the cell membrane and cell growth.

Inositol is effective in treating obsessive-compulsive disorder. Its effectiveness is comparable to that of selective serotonin reuptake inhibitors, or so-called non-tricyclic antidepressants, and is virtually free of side effects (Fux M. et al. Inositol treatment of obsessive-compulsive disorder. American Journal of Psychiatry, 1996 , 153: 1219-21).

Initially Nestler et al. 2000, and then more numerous and valid scientific evidences have been gathered demonstrating the usefulness of inositol, in particular of the isomer D-chiro-inositol, in the treatment of polycystic ovary syndrome (PCOS) (Nestler JE et al. Role inositolphosphoglycan of mediators of insulin action in the polycystic ovary syndrome. J Pediatr Endocrinol Metab, 2000, 13: 1295-8.

According to the present invention in the composition the concentration of inositol, preferably in the isomeric form of myo-inositol, ranges between 0.01% w/w and 1% w/w, preferably from 0.05% w/w to 0.5 % w/w, even more preferably the inositol concentration is 0.1% w/w.

In another embodiment of the present invention the composition comprising inositol, preferably in the isomeric form of myo-inositol, further comprises hypromellose.

The hypromellose, or hydroxypropyl methylcellulose, is a semisynthetic polymer, inert, viscoelastic, widely used as an additive in the food industry, as an excipient for the production of oral drugs, and as ophthalmic lubricant.

According to this embodiment in the composition the hypromellose concentration ranges between 0.03% w/w and 3% w/w, preferably from 0.15% w/w to 1.5% w/w, even more preferably the hypromellose concentration is 0.3% w/w.

The composition according to the invention is an aqueous solution, clear and colorless, with pH between 7.0 and 7.4, and an osmolarity between 250 and 300 mOsm/Kg.

The pH value is obtained by a buffering system suitable to maintain the pH value in the range between 7.0 and 7.4. Various buffering systems known to the skilled can be used in some embodiments of the present invention. However, in some preferred embodiments of the present invention the sodium phosphate monobasic/dibasic system providing adequate buffering capacity to maintain the pH in the desired range is used.

The osmolarity of the composition value is guaranteed by the presence of an adequate amount of acceptable salt, preferably the composition comprises sodium chloride in suitable amount to determine a value of osmolarity between 250 and 300 mOsm/Kg.

In addition to myo-inositol, or in addition to the association of myo-inositol and hydroxypropylmethylcellulose, the composition of the present invention may further comprise other stabilizing agents of the solution, lubricating agents, moisturizing agents, and structurally or functionally similar agents thereof. It is provided in the composition according to the invention also the presence preservatives and chelating agents.

The ophthalmic composition according to the present invention may include in its formulation also one or more additional therapeutic agents, such as growth factor, anti-inflammatory agents, vitamins, hyaluronic acid, fibronectin, collagen and amino acids.

The formulations of the present invention are prepared by mixing and stirring all the ingredients until all the components are in solution.

The solution thus obtained is then sterilized by filtration (0.2 micron), or by means of heat treatment at temperature of 121 °C, for 20 min, at a pressure of 1 atmosphere. However, the sterilization conditions may be varied and optimized by the skilled in the field.

### Pharmaceutical forms and doses of the composition according to the invention

### Pharmaceutical compositions

The ophthalmic composition according to the present invention can be prepared in any pharmaceutical form which allows the corneal application thereof, such as eye drops, collirium, gels, ointments, but preferably it is prepared in the form of eye drops.

There are now given for illustrative purposes two examples of particularly preferred formulation. Other aspects, advantages and modifications within the scope of the invention will be evident to the skilled in the relevant technical field.

### Example 1

| Ingredient | % w/w |
|---|---|
| Myo-inositol | 0.1 |
| Sodium chloride | 0.5 |
| Sodium monobasic phosphate dihydrate | 0.225 |
| Sodium dibasic phosphate dihydrate | 0.685 |
| Disodium EDTA | 0.1 |
| Sodium Hydroxymethylglycinate | 0.2 |
| Distilled water | q.s. to 100 g |

### Example 2

| Ingredient | % w/w |
|---|---|
| Myo-inositol | 0.1 |
| Hydroxypropyl methylcellulose | 0.3 |
| Sodium chloride | 0.5 |
| Sodium monobasic phosphate dihydrate | 0.225 |
| Sodium dibasic phosphate dihydrate | 0.685 |
| Disodium EDTA | 0.1 |
| Sodium hydroxymethylglycinate | 0.2 |
| distilled water | q.s. to 100 g |

The composition according to the invention, in its various embodiments, is administered in any amount and through any suitable route of administration for ocular treatment. The exact dosage will be chosen by the physician according to the patient's condition that has to be treated. Dosage and administration route are adjusted to provide sufficient levels of the active ingredient or to maintain the desired effect. Additional factors that may be considered include the severity of the condition, the course of the disease, age, weight and gender of the patient, time and frequency of administration, diet, combination with other drugs, sensitivity reactions, tolerance and response to therapy.

The composition according to the invention is formulated in a form suitable for topical application in the eye, preferably in the form of eye drops, each drop forming a dosage unit so as to allow easy administration and uniformity of dosage.

The treatment method of accommodative asthenopia according to the present invention is not limited in its use to Humans, but it can be extended, where appropriate, also to all Mammals.

The collyrium according to the present invention has proved to be highly tolerable by patients, who in clinical tests state a feeling of wellbeing following the use of the product, as reported below. In particular, the composition in the embodiment comprising only inositol as the active principle, has proved its effectiveness even without the addition of tear substitutes, often included in the formulations of similar products currently available on the market. This feature has allowed the development of a particularly simple formulation, which provides the least number of components and excipients, minimizing the risk of possible allergic or sensitization phenomena.

Furthermore, the composition according to the invention can be administrated in order to provide relief to the stressed eye following electrostimulation treatment in case of presbyopia. In fact, still in progress studies carried out by the Applicant have preliminarly shown that the ophthalmic composition of the invention exerts its activity also in prolonging the effects of treatment with micro-electrostimulation of the ciliary body in case of presbyopia. As described in European patent application EP 09762195.7, electrostimulation of the ciliary body is an effective treatment allowing to solve the condition of presbyopia. Ciliary muscle serves to focus on objects at varying distances. To obtain this result the muscle continually modifies the crystalline shape that, as the lens of a camera, is able to see at different distances. By passing the time, and usually after the age of 40, this muscle gradually loses its efficiency and the use of reading glasses becomes necessary. The same glasses overcome and make completely useless the muscle function, thus the muscle gradually atrophies. The electrostimulation of the ciliary muscle is able to restore the functioning of the muscle from the first session and more and more in the subsequent sessions allowing to return to read up without glasses. Patients better responding to this new therapies are those that do not have important visual defects relating farsightedness and/or myopia, and who are aged between 40 and 50 years. The treatment is carried out by applying a scleral contact lens with four electrodes positioned in correspondence of the muscle. The lens is then connected to an electrical stimulator which transmits small electrical pulses to passively stimulating muscle contraction. A stimulation session of the ciliary muscle has a duration of about 8 minutes. After 12 hours the patient starts to read without glasses. It should be remembered that being a passive exercise muscle electrostimulation must be periodically repeated according to customized schemes depending on muscle strength and age of the subject. The use of the composition according to the invention subsequently to the electrostimulation treatment allows to lengthen the period of effectiveness of the treatment by decreasing the need for frequent stimulation interventions.

### Clinical study 1

In order to demonstrate the effectiveness of the composition according to the invention in the embodiment comprising only inositol as active ingredient of the formulation according to example 1, said composition has been instilled in patients with defects of refraction not greater than 1.5 measured in diopters spherical equivalent.

In the study 30 patients aged between 41 and 51 years were enrolled. Patients were divided into three groups:
Group A: n = 10 patients, aged between 41 and 44 years (6 men, 4 women);
Group B: n = 10 patients, aged between 45 and 48 years (5 men, 5 women);
Group C: n = 10 patients, aged between 49 and 51 years old (3 men, 7 women).

Patients preliminarily underwent a thorough eye exam, in both eyes, which included:
Examination of the natural and correct visual acuity at 6 meters distance.

Refraction in miosis and cycloplegia with autorefractometer Nidek AR1
Examination at the slit lamp
Fundus examination (Volk lens)
Tonometry with Goldmann tonometer

Accommodative amplitude measurement without correction with autrorefractometer Nidek AR1.

All selected patients presented no ocular pathology and corrected visual acuity of 10/10 in both eyes.

All patients were subsequently treated with the eye drops composition having formulation as in Example 1 for a period of thirty consecutive days. Patients received 3 drops 3 times a day: in the morning upon waking, half day (about 15-16 pm) and at night before sleeping. At intervals of 10 days accommodative amplitude measurement was performed and any subjective side effect was noted; thirty days after all patients were subjected to full eye exam.

The accommodative amplitude measurements of the three groups at 10, 20 and 30 days are reported in Tables 1, 2 and 3.

### Results

Tables 1, 2, and 3 show the accommodative changes before, during and at the end of the treatment with the composition comprising inositol as main active ingredient. The accommodative amplitude was measured in diopters.

**Table 1 - Patients aged between 41 and 44 years before and after treatment**

| Patient | age | pretreatment accomodative amplitude | 10 days accomodative amplitude | 20 days accomodative amplitude | 30 days accomodative amplitude |
|---|---|---|---|---|---|
| 1 | 41 | 4.15 | 4.22 | 4.25 | 4.22 |
| 2 | 41 | 3.80 | 3.96 | 4.02 | 4.08 |
| 3 | 42 | 3.80 | 3.79 | 3.77 | 3.88 |
| 4 | 42 | 3.98 | 3.90 | 3.98 | 4.03 |
| 5 | 42 | 4.20 | 4.19 | 4.22 | 4.15 |
| 6 | 42 | 3.67 | 3.36 | 4.04 | 4.12 |
| 7 | 43 | 3.87 | 3.90 | 3.75 | 3.88 |
| 8 | 43 | 3.72 | 3.74 | 3.77 | 4.10 |
| 9 | 43 | 3.84 | 3.90 | 3.96 | 4.05 |
| 10 | 44 | 3.88 | 3.90 | 3.75 | 3.34 |

**Table 2 - Patients aged between 45 and 48 years before and after treatment**

| Patient | age | pretreatment accomodative amplitude | 10 days accomodative amplitude | 20 days accomodative amplitude | 30 days accomodative amplitude |
|---|---|---|---|---|---|
| 1 | 45 | 3.35 | 3.12 | 3.32 | 3.32 |
| 2 | 45 | 3.22 | 3.12 | 2.99 | 3.12 |
| 3 | 45 | 3.32 | 3.32 | 3.41 | 3.44 |
| 4 | 46 | 2.88 | 3.03 | 3.12 | 2.99 |
| 5 | 46 | 2.77 | 3.10 | 3.12 | 2.76 |
| 6 | 46 | 2.44 | 3.11 | 3.22 | 3.26 |
| 7 | 47 | 2.53 | 2.74 | 2.83 | 3.31 |
| 8 | 47 | 2.15 | 2.63 | 2.73 | 2.88 |
| 9 | 47 | 2.13 | 2.53 | 2.58 | 2.68 |
| 10 | 48 | 2.10 | 2.31 | 2.42 | 2.34 |

**Table 3 - Patients aged between 49 and 51 years before and after treatment**

| Patient | age | pretreatment accomodative amplitude | 10 days accomodative amplitude | 20 days accomodative amplitude | 30 days accomodative amplitude |
|---|---|---|---|---|---|
| 1 | 49 | 2.48 | 2.55 | 2.59 | 2.62 |
| 2 | 49 | 2.36 | 2.25 | 2.41 | 2.45 |
| 3 | 50 | 1.89 | 1.89 | 1.89 | 1.99 |
| 4 | 50 | 1.76 | 1. 87 | 1.92 | 1.94 |
| 5 | 50 | 1. 68 | 1.78 | 1.77 | 1.61 |
| 6 | 50 | 1.69 | 1.78 | 1.72 | 1.73 |
| 7 | 51 | 1.64 | 1.88 | 1.65 | 1.66 |
| 8 | 51 | 1.53 | 1.83 | 1.45 | 1.50 |
| 9 | 51 | 1.57 | 1.81 | 1.49 | 1.55 |
| 10 | 51 | 1.45 | 1.61 | 1.45 | 1.50 |

The results show a substantial, although moderate, improvement in accommodative amplitude. This improvement was clearly perceived by patients who reported subjectively a decrease of accommodative asthenopia especially after the first 10 days of treatment and for the entire duration of the administration. The differences between the mean pre- and post-treatment value after 30 days treatment show and improvement in accommodative amplitude of 4.1%.

The instillation of the composition having formulation shown in Example 1 resulted only in certain subjects transients and subjective side effects, such as temporary redness after administration the eye drops, temporary dryness, burning. The phenomena occurred about 5 minutes after the administration and persisted for several minutes, however they disappeared after the administration of artificial tears. Nevertheless, it should be considered that the incidence of dry eye side-effects reported by some subjects could be related to the age of the patients involved in the study, in fact, the so-called syndrome of "dry eye" is very common after 40 years of age regardless of race, more frequently in women than in men, rather than due to real side effects due to the composition administration.

At the end of the thirty-day clinical trial all enrolled patients were examined according to the initial protocol: none has shown signs of intolerance or eye diseases. The natural and correct farsightedness remained unchanged.

### Clinical study 2

The effectiveness of the composition according to the invention in the embodiment comprising the association of inositol and hypromellose as active principle, i.e. the formulation according to example 2, has been assessed in a clinical trial similar to that previously described, wherein said composition has been instilled in patients with defects of refraction not greater than 1.5 diopters measured in spherical equivalent.

In the study 30 patients aged between 41 and 51 years were enrolled. Patients were divided into three groups:
Group A: n = 10 patients, aged between 41 and 44 years (7 men, 3 women);
Group B: n = 10 patients, aged between 45 and 48 years (6 men, 4 women);
Group C: n = 10 patients, aged between 49 and 51 years (2 men, 8 women).

Patients preliminarily underwent a thorough eye exam, in both eyes, which included:
Examination of the natural and correct visual acuity at 6 meters distance.

Refraction in miosis and cycloplegia with autorefractometer Nidek AR1
Examination at the slit lamp
Fundus examination (Volk lens)
Tonometry with Goldmann tonometer

Accommodative amplitude measurement without correction with autrorefractometer Nidek AR1.

All selected patients presented no ocular pathology and corrected visual acuity of 10/10 in both eyes.

All patients were subsequently treated with the eye drops composition having formulation as in Example 2 for a period of thirty consecutive days. Patients received 3 drops 3 times a day: in the morning upon waking, half day (about 15-16 pm) and at night before sleeping. At intervals of 10 days accommodative amplitude measurement was performed and any subjective side effect was noted, thirty days after all patients were subjected to full eye exam.

The accommodative amplitude measurements of the three groups at 10, 20 and 30 days are reported in Tables 4, 5 and 6.

### Results

Tables 4, 5, and 6 show the accommodative changes before, during and at the end of the treatment with the composition comprising inositol and ipromellose as main active ingredient. The accommodative amplitude was measured in diopters.

**Table 4 - Patients aged between 41 and 44 years before and after treatment**

| Patient | age | pretreatment accomodative amplitude | 10 days accomodative amplitude | 20 days accomodative amplitude | 30 days accomodative amplitude |
|---|---|---|---|---|---|
| 1 | 41 | 4.20 | 4.18 | 4.21 | 4.20 |
| 2 | 41 | 3.88 | 3.92 | 4.02 | 4.06 |
| 3 | 42 | 3.89 | 3.78 | 3.77 | 3.88 |
| 4 | 42 | 3.92 | 3.95 | 3.98 | 4.03 |
| 5 | 42 | 4.05 | 4.12 | 4.10 | 4.15 |
| 6 | 42 | 3.51 | 3.44 | 4.01 | 4.12 |
| 7 | 43 | 3.67 | 3.57 | 3.66 | 3.88 |
| 8 | 44 | 3.72 | 3.88 | 3.90 | 4.10 |
| 9 | 44 | 3.79 | 3.92 | 3.98 | 4.05 |
| 10 | 44 | 3.12 | 3.10 | 3.22 | 3.34 |

**Table 5 - Patients aged between 45 and 48 years before and after treatment**

| Patient | age | pretreatment accomodative amplitude | 10 days accomodative amplitude | 20 days accomodative amplitude | 30 days accomodative amplitude |
|---|---|---|---|---|---|
| 1 | 45 | 3.30 | 3.12 | 3.36 | 3.32 |
| 2 | 46 | 3.01 | 2.99 | 2.99 | 3.12 |
| 3 | 46 | 3.12 | 3.16 | 3.41 | 3.44 |
| 4 | 46 | 2.88 | 3.01 | 3.12 | 2.99 |
| 5 | 46 | 2.77 | 3.12 | 3.14 | 2.89 |
| 6 | 46 | 2.98 | 3.16 | 3.22 | 3.02 |
| 7 | 47 | 2.12 | 2.77 | 2.88 | 2,89 |
| 8 | 48 | 2.16 | 2.69 | 2.89 | 2.56 |
| 9 | 48 | 2.01 | 2.54 | 2.67 | 2.34 |
| 10 | 48 | 1, 99 | 2.36 | 2.51 | 2.13 |

**Table 6 - Patients aged between 49 and 51 years before and after treatment**

| Patient | age | pretreatment accomodative amplitude | 10 days accomodative amplitude | 20 days accomodative amplitude | 30 days accomodati ve amplitude |
|---|---|---|---|---|---|
| 1 | 50 | 2.44 | 2.55 | 2.59 | 2.51 |
| 2 | 50 | 2.32 | 2.25 | 2.41 | 2.40 |
| 3 | 50 | 1.88 | 1.85 | 1.89 | 1.99 |
| 4 | 50 | 1.75 | 1.82 | 1.92 | 1.91 |
| 5 | 50 | 1.67 | 1.73 | 1.77 | 1.77 |
| 6 | 50 | 1.67 | 1.70 | 1.72 | 1. 84 |
| 7 | 51 | 1. 60 | 1.64 | 1.65 | 1.64 |
| 8 | 51 | 1.55 | 1.55 | 1.45 | 1.50 |
| 9 | 51 | 1.50 | 1.49 | 1.49 | 1.55 |
| 10 | 51 | 1.45 | 1.55 | 1.45 | 1.50 |

Albeit without statistical significance the results show a substantial improvement of the accommodative amplitude also obtained with the composition according to the invention having formulation as shown in Example 2. This improvement was clearly perceived by patients who reported subjectively a decrease of accommodative asthenopia especially after the first 10 days of treatment and for the entire duration of the administration. The differences between the mean pre-and post-treatment value after 30 days treatment show and improvement in accommodative amplitude of 4.5%.

The instillation of the composition having formulation shown in Example 2 resulted only in certain subjects transients and subjective side effects, such as temporary redness after administration the eye drops, temporary dryness, burning. At the end of the thirty days treatment period, all enrolled patients were reviewed according to the initial protocol without showing any signs of intolerance or eye diseases. The natural and correct farsightness remained unchanged.

### Conclusions

Accommodative asthenopia is a multifactorial phenomenon related to the progressive decrease in accommodative amplitude of the ciliary muscle, but also to the type of close work activity, its duration in the day and in the time, and to the work environment as well. The use of the ophthalmic composition of the invention based on myo-inositol, and furthermore in the presence of hypromellose, causes, without any side effect, improvement of the accommodative amplitude and a prolongation of the effect of the therapy obtained by means of microelectrostimolation of the ciliary muscle against the presbyopia.

## Claims

1. An ophthalmic composition **characterized by** the fact to comprise myo-inositol at a concentration ranging between 0.01 %w/w and 0.4% w/w.

2. The ophthalmic composition according to claim 1, **characterized in that** the myo-inositol concentration is 0.1 %w/w.

3. The ophthalmic composition according to claims 1 and 2, further comprising hydroxypropylmethylcellulose.

4. The ophthalmic composition according to claim 3, **characterized in that** the hydroxypropylmethylcellulose concentration ranges between 0.03% w/w and 3% w/w.

5. The ophthalmic composition according to claim 4, **characterized in that** the hydroxypropylmethylcellulose concentration is 0.3% w/w.

6. The ophthalmic composition according to anyone of claims 1-5, **characterized in that** it has pH value between 7.0 and 7.4, and an osmolarity value between 250 and 300 mOsm/kg.

7. The ophthalmic composition according to anyone of claims 1-6, further comprising stabilizing agents of the solution, lubricating agents, moisturizing agents, preservatives and chelating agents.

8. The ophthalmic composition according to anyone of claims 1 to 7, further comprising growth factors, anti-inflammatory agents, vitamins, hyaluronic acid, fibronectin, collagen and amino acids.

9. The ophthalmic composition according to any one of the preceding claims **characterized in that** it is in any form allowing corneal application.

10. The ophthalmic composition according to claim 9 in the form of: eye drops, gels, ointments, collirium.

11. The ophthalmic composition according to any one of the preceding claims **characterized in that** it has the following formulation:
| | |
|---|---|
| Myo-inositol | 0.1% w/w |
| Sodium chloride | 0.5% w/w |
| Sodium phosphate monobasic dihydrate | 0.225% w/w |
| Sodium phosphate dibasic dihydrate | 0.685% w/w |
| Disodium EDTA | 0.1% w/w |
| Sodium hydroxymethylglycinate | 0.2% w/w |
| Distilled water | q.s. to 100 g. |

12. The ophthalmic composition according to any one of the preceding claims **characterized in that** it has the following formulation:
| | |
|---|---|
| Myo-inositol | 0.1% w/w |
| Hydroxypropyl methylcellulose | 0.3% w/w |
| Sodium chloride | 0.5% w/w |
| Sodium phosphate monobasic dihydrate | 0.225% w/w |
| Sodium phosphate dibasic dihydrate | 0.685% w/w |
| Disodium EDTA | 0.1% w/w |
| Sodium hydroxymethylglycinate | 0.2% w/w |
| Distilled water | q.s. to 100 g. |

13. Ophthalmic composition as defined in claims 1 to 12 for the use in the treatment of eye disorders caused by environmental stress, mechanical, visual, including asthenopia and accommodative effort.

14. Ophthalmic composition as defined in claims 1 to 12 for use in the treatment following to the electrostimulation of the ciliary muscle against presbyopia.
